(19) Europäisches Patentamt
European Patent Office
Office européen des brevets

(11) Veröffentlichungsnummer: **0 174 624**
**B1**

(12) # EUROPÄISCHE PATENTSCHRIFT

(45) Veröffentlichungstag der Patentschrift: **22.07.87**

(51) Int. Cl.⁴: **C 07 C 69/68,** C 07 C 67/00

(21) Anmeldenummer: **85111340.7**

(22) Anmeldetag: **07.09.85**

(54) **Verfahren zur Herstellung von Milchsäureestern.**

(30) Priorität: **12.09.84 DE 3433400**

(43) Veröffentlichungstag der Anmeldung: **19.03.86 Patentblatt 86/12**

(45) Bekanntmachung des Hinweises auf die Patenterteilung: **22.07.87 Patentblatt 87/30**

(84) Benannte Vertragsstaaten: **BE CH DE FR GB IT LI NL**

(56) Entgegenhaltungen:
**DE-A-3 222 837**
**US-A-1 668 806**
**US-A-1 695 449**
**US-A-2 390 140**

(73) Patentinhaber: **BASF Aktiengesellschaft, Carl-Bosch- Strasse 38, D-6700 Ludwigshafen (DE)**

(72) Erfinder: **Bott, Kaspar, Dr., Rieslingweg 4, D-6706 Wachenheim (DE)**
Erfinder: **Boehm, Walter, Dr., Mittlerer Waldweg 11, D-6719 Kirchheim (DE)**
Erfinder: **Fritz, Gerhard, Dr., Limburgstrasse 23, D-6701 Dannstadt- Schauernheim (DE)**
Erfinder: **Martin, Christoph, Dr., Kolpingstrasse 6, D-6800 Manheim 1 (DE)**
Erfinder: **Siegel, Hardo, Dr., Hans- Purrmann- Allee 25, D-6720 Speyer (DE)**

**EP 0 174 624 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Herstellung optisch reiner D- oder L-Milchsäurealkylester, indem man fermentativ hergestelltes Calciumlactat mit Alkoholen in Gegenwart einer starken Säure umsetzt.

Die fermentative Darstellung optisch reiner Milchsäure ist aus EP 0 069 291 oder Ind. Eng. Chem. 44, 1958 (1952) bekannt. Sie wird in Gegenwart von Calciumcarbonat durchgeführt, so daß nach beendeter Reaktion eine wäßrige Lösung oder Suspension anfällt, welche 6 bis 20 % Calciumlactat neben verschiedenen organischen oder anorganischen Verunreinigungen enthält, die entweder als Zusatzstoffe zur Fermentation zugegeben wurden oder Stoffwechselprodukte sind, die während der Fermentation entstehen. Ferner sind als Feststoffe noch Biomasse und ggf. nicht verbrauchtes Calciumcarbonat vorhanden.

Anschließend wird die Milchsäure aus dem Calciumsalz freigesetzt, indem man mit Schwefelsäure ansäuert, wobei Gips ausfällt, den man zusammen mit der Biomasse abfiltert. Als Filtrat erhält man eine wäßrige Milchsäurelösung mit einem Gehalt von ca. 10 % Milchsäure, die noch verschiedene gelöste Verunreinigungen enthält. Diese Lösung wird in der Regel aufkonzentriert und geeigneten Reinigungsoperationen unterworfen.

Die betriebene Verfahrensweise ist mit verschiedenen Nachteilen verbunden. So fallen erhebliche Mengen an Calciumsulfat an, das aufgrund der darin enthaltenen Biomasse zu Nachgärungen neigt und deshalb nicht ohne Nachbehandlung deponiert werden kann. Eine Verwertung z.B. als Baustoff ist wegen ungenügender Reinheit nicht möglich. Nachteilig ist auch, daß durch das Nachwaschen des Filterkuchens eine weitere Verdünnung der wäßrigen Milchsäurelösung stattfindet, wodurch die Isolierung des Produktes, sei es die Milchsäure oder, falls bei der Freisetzung aus dem Calciumlactat ein Alkohol zugesetzt wird, der Milchsäureester, erschwert wird. Schließlich bereitet das Aufkonzentrieren der wäßrigen Milchsäurelösung z.B. durch destillative Entfernung des Wassers häufig Schwierigkeiten, da Reste der Biomasse und insbesondere gelöstes Calciumsulfat, das zunehmend ausfällt, zu Verkrustungen in dem Apparaturen führen. Weiterhin geht ein geringer Anteil der Milchsäure mit dem Wasser verloren.

Der Erfindung lag daher die Aufgabe zugrunde, ein Verfahren zur Veresterung einer durch Fermentation hergestellten optisch reinem Milchsäure zu entwickeln, das die geschilderten Nachteile umgeht und den Ester in guter Ausbeute und hoher optischer Reinheit liefert.

Demgemäß wurde ein Verfahren zur Herstellung optisch reiner D- oder L-Milchsäurealkylester durch Umsetzung von fermentativ hergestelltem Calciumlactat mit Alkoholen in Gegenwart einer starken Säure gefunden, welches dadurch gekennzeichnet ist, daß man das rohe Fermentationsgemisch filtriert, aus dem Filtrat das Calciumlactat durch Sprühtrocknung als Feststoff isoliert und es in Gegenwart einer Säure, die ein gut wasserlösliches Calciumsalz bildet, mit Alkoholen umsetzt, wobei das in Reaktionsgemisch vorliegende bzw. bei der Veresterung gebildete Wasser durch azeotrope Destillation mit Hilfe eines Schleppmittels ausgekreist und der Milchsäureester in an sich bekannter Weise aus den Reaktionsgemisch isoliert wird.

Das der Fermentation entstammende rohe Reaktionsgemisch, das die D- oder L-Milchsäure als Calciumlactat enthält, wird zur Abtrennung von Feststoffen wie Biomasse oder nicht verbrauchtem Calciumcarbonat filtriert. Dabei ist darauf zu achten, daß die Temperatur der Lösung so hoch gehalten wird, daß kein Calciumlactat ausfällt. In der Regel wird bei Temperaturen von 50 bis 90, insbesondere 60 bis 80°C gearbeitet. Die Filtration erfolgt nach den üblichen Techniken, z.B. unter Verwendung von Filterpressen und gegebenenfalls in Gegenwart von Filterhilfsmitteln wie Kieselgel.

Anschließend wird das Filtrat in einem Sprühturm mittels Luft als Wärme, überträger getrocknet. Dabei beträgt die Lufteingangstemperatur 280 bis 300°C und die Luftausgangstemperatur 110°C. Das Gelingen dieses Aufarbeitungsschrittes ist überraschend, da man annehmen mußte, daß das Rohcalciumlactat aufgrund der darin enthaltenen Verunreinigungen verkleben würde und somit nicht rieselfähig sein könnte.

Weiterhin hätte man erwartet, daß bei den hohen Temperaturen der Sprühtrocknung das optisch reine Lactat wegen der Beweglichkeit des "α-Wasserstoffatoms" eine zumindest teilweise Racemisierung erleiden würde.

Tatsächlich wird keine nennenswerte Konfigurationsumkehr am Asymmetriezentrum beobachtet.

Zur Veresterung wird das Calciumlactat mit niedermolekularen Alkoholen ROH in Gegenwart einer starken Säure umgesetzt. Die Alkohole können verzweigt oder unverzweigt sein und 1 bis 10, insbesondere 1 bis 5 Kohlenstoffatome enthalten. Vorteilhaft wird die Reaktion mit sekundären und primären Alkoholen durchgeführt, also z.B. mit Ethanol, Propanol, Butanol, Isobutanol, Pentanol und Isopentanol. Auch mehrwertige Alkohole wie Glykol oder Glycerin können verwendet werden.

Als starke Säuren kommen solche in Frage, die ein gut wasserlösliches Calciumsalz bilden und als Veresterungskatalysator wirksam sein können. Hier sind Salpetersäure, HBr und Sulfonsäuren zu nennen, besonders geeignet ist Salzsäure, die vorteilhaft in konzentrierter Form verwendet wird.

Damit ergibt sich folgende Reaktionsgleichung:

$$(H_3C-\underset{OH}{\overset{H}{C^*}}-COO)_2Ca + 2\ ROH + 2\ HCl \rightleftharpoons H_3C-\underset{OH}{\overset{H}{C^*}}-COOR + 2\ H_2O + CaCl_2$$

Um das Veresterungsgleichgewicht nach rechts zu verschieben, wird bekanntlich eine der Ausgangskomponenten, meistens die billigere, im Überschuß eingesetzt, während die Umsetzungsprodukte dem Reaktionsgemisch entzogen werden.

Für das erfindungsgemäße Verfahren ist es wichtig, daß der Alkohol im Überschuß verwendet wird und zwar nicht nur wegen der Verschiebung des o.a. Gleichgewichts, sondern um die Konzentration der in Gleichgewicht auftretenden dimeren und trimeren Milchsäureester, die durch Reaktion der Milchsäure mit der Hydroxylgruppe eines weiteren Moleküls Milchsäure entstehen, möglichst klein zu halten. In der Regel werden 2 bis 4 mol Alkohol pro Mol Milchsäure verwendet.

Um das mit den Reaktionskomponenten eingeschleppte Wasser, sei es in Form von wäßriger Salzsäure oder als wasserhaltiges Calciumlactat, das nach der Sprühtrocknung noch einen Wasseranteil von 0 bis 25 % aufweisen kann, und das bei der Veresterung entstandene Wasser den Reaktionsgemisch zu entziehen, wird es in Gegenwart eines Schleppmittels wie Cyclohexan oder Toluol azeotrop abdestilliert.

Bekanntlich bilden höhere Alkohole mit Kohlenstoffzahlen von 4 und >4 selbst Azeotrope mit Wasser, so daß sich in bestimmten Fällen die Schleppmittelzugabe erübrigt. In dem vorliegenden Fall wird aber die Schleppwirkung der betreffenden Alkohole durch das anwesende $CaCl_2$ stark herabgesetzt. Für das erfindungsgemäße Verfahren ist es deshalb wichtig, daß auch in diesen Fällen, z.B. bei Verwendung von Isobutanol ein Schleppmittel zugesetzt wird, um nicht beträchtliche Ausbeuteverluste an Wertprodukt durch einen niedrigen Veresterungsgrad hinnehmen zu müssen.

Zur Isolierung des Wertproduktes wird das nach der Veresterung anfallende Reaktionsgemisch mit Wasser behandelt, wobei eine salzfreie organische Phase und eine salzreiche wäßrige Phase anfällt. Die Extraktion kann nach den dafür üblichen Techniken kontinuierlich oder diskontinuierlich durchgeführt werden.

Anschließend wird die organische Phase neutralisiert, z.B. mit verdünnter Natronlauge, und der Milchsäureester durch Destillation nach den dafür üblichen Techniken, vorteilhaft bei vermindertem Druck, gereinigt. Die Neutralisation vor der destillativen Reinigung ist zweckmäßig, um eine nachträgliche Bildung höherer Milchsäureester (Dimere, Trimere) zu vermeiden.

Nach dem erfindungsgemäßen Verfahren wird der Milchsäureester in guten Ausbeuten und hoher optischer Reinheit erhalten.

Optisch aktive Milchsäureester sind wichtige Zwischenprodukte zur Darstellung optisch aktiver Herbizide.

**Beispiel**

Eine durch Fermentation hergestellte Calciumlactatlösung wurde bei 70°C in Gegenwart von Kieselgel als Filterhilfsmittel in einer Filterpresse filtriert und anschließend heiß auf einen Sprühturm gegeben, wobei die Lufteingangstemperatur 280°C und die Luftausgangstemperatur 110°C betrug. 105 Teile des anfallenden Roh-Calciumlactats, das 67 % Milchsäure enthielt, wurde mit 203 Teilen Isobutanol, 137 l Toluol und 97 Teile konzentrierter Salzsäure versetzt und 5 Stunden an Rückfluß erhitzt. Dabei wurden 82 Teile Wasser ausgekreist. Nach Abkühlen auf Raumtemperatur werden 103 Teile Wasser zugesetzt und die wäßrige, calciumchloridhaltige Phase, ca 154 Teile, abgetrennt.

Die organische Phase wurde mit 50 %iger Natronlauge neutralisiert und anschließend destillativ aufgearbeitet.

Die Ausbeute an optisch aktivem Milchsäureisobutylester lag bei 89 %, entsprechend 120 Teilen, bezogen auf Calciumlactat. Die optische Reinheit des Produktes betrug > 95 %.

**Patentansprüche**

1. Verfahren zur Herstellung optisch reiner D- oder L-Milchsäurealkylester durch Umsetzung von fermentativ hergestelltem Calciumlactat mit Alkoholen in Gegenwart einer starken Säure, dadurch gekennzeichnet, daß man das rohe Fermentationsgemisch heiß filtriert, aus den Filtrat das Calciumlactat durch Sprühtrocknung als Feststoff isoliert und es in Gegenwart einer Säure, die ein gut wasserlösliches Calciumsalz bildet, mit Alkoholen umsetzt, wobei das in Reaktionsgemisch vorliegende bzw. bei der Veresterung gebildete Wasser durch azeotrope Destillation mit Hilfe eines Schleppmittels ausgekreist und der Milchsäureester in an sich bekannter Weise aus den Reaktionsgemisch isoliert wird.

2. Verfahren nach Anspruch 1, dadurch gekennzeichnet, daß man die Veresterung in Gegenwart von Salzsäure durchführt.

3. Verfahren nach Anspruch 1, dadurch gekennzeichnet, daß man niedermolekulare verzweigte oder

unverzweigte Alkohole mit 1 bis 10, vorzugsweise 1 bis 5 Kohlenstoffatome verwendet.

4. Verfahren nach Anspruch 1, dadurch gekennzeichnet, daß man den Alkohol in Mengen von 1 bis 5 mol pro Mol Milchsäure zusetzt.

5. Verfahren nach Anspruch 1, dadurch gekennzeichnet, daß man die Filtration bei Temperaturen von 50 bis 90, insbesondere 60 bis 80° C durchführt.

6. Verfahren nach Anspruch 1, dadurch gekennzeichnet, daß bei der Sprühtrocknung die Lufteingangstemperatur 280 bis 300° C und die Luftausgangstemperatur 110° C beträgt.

**Claims**

1. A process for the preparation of an optically pure alkyl D- or L-lactate by reaction of calcium lactate, prepared by fermentation, with an alcohol in the presence of a strong acid, wherein the crude fermentation mixture is filtered while hot, the calcium lactate is isolated as a solid from the filtrate by spray drying and is reacted with an alcohol in the presence of an acid which forms a readily water-soluble calcium salt, the water present in the reaction mixture or formed during the esterification is separated off by azeotropic distillation with the aid of an entraining agent, and the lactic acid ester is isolated from the reaction mixture in a conventional manner.

2. A process as claimed in claim 1, wherein the esterification is carried out in the presence of hydrochloric acid.

3. A process as claimed in claim 1, wherein a branched or straight-chain low molecular weight alcohol of 1 to 10, preferably 1 to 5, carbon atoms is used.

4. A process as claimed in claim 1, wherein the alcohol is added in an amount of from 1 to 5 moles per mole of lactic acid.

5. A process as claimed in claim 1, wherein filtration is carried out at from 50 to 90° C, in particular from 60 to 80° C.

6. A process as claimed in claim 1, wherein, in the spray-drying operation, the temperature of the incoming air is from 280 to 300° C and that of the outgoing air is 110° C.

**Revendications**

1. Procédé de préparation d'esters alkyliques d'acide D- ou L-lactique optiquement purs par réaction de lactate de calcium préparé par fermentation avec des alcools en présence d'un acide fort, caractérisé en ce qu'on filtre à chaud le mélange de fermentation brut, on isole du filtrat le lactate de calcium sous forme de matière solide par séchage par pulvérisation et on le fait réagir avec des alcools en présence d'un acide qui forme un sel de calcium bien soluble dans l'eau, l'eau présente dans le mélange réactionnel ou formée lors de l'estérification étant éliminée par distillation azéotrope à l'aide d'un agent d'entraînement et l'ester d'acide lactique étant isolé du mélange réactionnel de façon connue en soi.

2. Procédé selon la revendication 1, caractérisé en ce qu'on procède à l'estérification en présence d'acide chlorhydrique.

3. Procédé selon la revendication 1, caractérisé en ce qu'on utilise des alcools à 1-10, de préférence à 1-5 atomes de carbone, à chaîne ramifiée ou non ramifiée, de faible poids moléculaire.

4. Procédé selon la revendication 1, caractérisé en ce qu'on ajoute l'alcool dans des proportions de 1 à 5 mol par mol d'acide lactique.

5. Procédé selon la revendication 1, caractérisé en ce qu'on procède à la filtration à des températures de 50 à 90° C, en particulier de 60 à 80° C.

6. Procédé selon la revendication 1, caractérisé en ce que lors du séchage par pulvérisation, la température de l'air à l'entrée est comprise entre 280 et 300° C et la température de l'air à la sortie s'élève à 110° C.